# EUROPEAN PATENT APPLICATION

(11) **EP 4 692 859 A1**
(43) Date of publication of application: **11.02.2026**
(21) Application number: 24796473.7
(22) Date of filing: 18.01.2024
(51) Int. Cl.: G01T 7/00, A61B 6/42, H04N 25/70

(54) **RADIATION IMAGING DEVICE**

(30) Priority: 27.04.2023 JP 2023073485
(71) Applicant: Hamamatsu Photonics K.K., Hamamatsu-shi, Shizuoka 435-8558 (JP)
(72) Inventor: TOYODA Kenta, Hamamatsu-shi, Shizuoka 435-8558 (JP); IWATA Takuya, Hamamatsu-shi, Shizuoka 435-8558 (JP); NAMBA Shuhei, Hamamatsu-shi, Shizuoka 435-8558 (JP); FUKAMIZU Seiji, Hamamatsu-shi, Shizuoka 435-8558 (JP)
(74) Representative: Hoffmann Eitle
(86) International application number: PCT/JP2024/001320
(87) International publication number: WO 2024/224714

(57) **Abstract**

A radiation imaging device according to an embodiment includes a sensor panel, a support member that supports the sensor panel, a control board disposed at a position facing a back surface of the support member, a flexible printed circuit disposed to connect the sensor panel and the control board, an IC chip mounted on the flexible printed circuit, and a heat absorbing member provided between the support member and the IC chip and configured to absorb heat from the IC chip. The heat absorbing member includes a heat absorbing portion disposed in a region overlapping the flexible printed circuit and having a contact surface that contacts a surface of the IC chip, and a heat radiating portion connected to the heat absorbing portion and extending outward beyond an outer edge of the flexible printed circuit. A thickness of the heat radiating portion is greater than a thickness of the heat absorbing portion.

## Description

### Technical Field

The present disclosure relates to a radiation imaging device.

### Background Art

A radiation imaging device is known that includes a sensor panel (radiation detection panel) supported by a support member, a control board (signal processing board) disposed on a side opposite to the sensor panel with the support member interposed therebetween, and a flexible printed circuit disposed so as to go around a side of the support member to connect the sensor panel and the control board (see, for example, Patent Literatures 1 and 2). Patent Literature 1 discloses a configuration in which a fixing material (for example, silicone gel, urethane gel, acrylic gel, or the like) having adhesiveness and excellent thermal conductivity is disposed between an IC chip provided on a flexible printed circuit and a support member. Patent Literature 2 discloses a configuration in which an IC chip provided on a flexible printed circuit and a support member (support base) are closely fixed by a fixing material (adhesive or adhesive tape) having heat dissipating properties and adhesiveness.

### Citation List

### Patent Literature

[Patent Literature 1] Japanese Unexamined Patent Application Publication No. 2014-025846
[Patent Literature 2] Japanese Unexamined Patent Application Publication No. 2002-131437

### Summary of Invention

### Technical Problem

According to the configurations disclosed in Patent Literatures 1 and 2, heat generated in the IC chip can be released (transmitted) to the support member via the fixing material. However, the heat transmitted to the support member is also transmitted to the sensor panel via the support member, and as a result, noise due to heat is generated in the sensor panel, which may cause deterioration in the image quality of a radiation image obtained by the radiation imaging device and a decrease in its reliability.

An object of an aspect of the present disclosure is to provide a radiation imaging device that can improve reliability.

### Solution to Problem

The present disclosure includes the radiation imaging devices of [1] to [12].
[1] A radiation imaging device including: a sensor panel provided with a detection region for detecting radiation; a support member having a support surface that supports the sensor panel, and a back surface opposite to the support surface; a control board disposed at a position facing the back surface of the support member and configured to process a signal read out from the sensor panel; a flexible printed circuit disposed to connect the sensor panel and the control board through a side of the support member when viewed from a first direction orthogonal to the support surface; an IC chip mounted on the flexible printed circuit and configured to perform processing for reading out the signal from the sensor panel; and a heat absorbing member provided between the support member and the IC chip on a side opposite to a side where the sensor panel is located with respect to the support member, and configured to absorb heat from the IC chip, wherein the heat absorbing member includes: a heat absorbing portion disposed in a region overlapping the flexible printed circuit when viewed from the first direction, and having a contact surface that contacts a surface of the IC chip opposite to a mounting surface of the IC chip on the flexible printed circuit; and a heat radiating portion connected to the heat absorbing portion and extending outward beyond an outer edge of the flexible printed circuit in a third direction intersecting a second direction, which is an extending direction of the flexible printed circuit, when viewed from the first direction, and wherein a thickness of the heat radiating portion in the first direction is greater than a thickness of the heat absorbing portion in the first direction.
   In the configuration of [1], the heat absorbing member includes the heat absorbing portion, which is disposed so as to overlap the flexible printed circuit and efficiently absorbs heat from the IC chip via the contact surface, and the heat radiating portion, which is formed to extend outward (in the third direction) from the heat absorbing portion. By providing the heat radiating portion in addition to the heat absorbing portion, and by providing the heat radiating portion, which is thicker than the heat absorbing portion, at a position that does not overlap the flexible printed circuit in the first direction, the overall volume and heat capacity of the heat absorbing member can be effectively increased, and the surface area of the portion of the heat radiating portion that is in contact with air can be appropriately secured. This allows heat from the IC chip to be effectively absorbed by the heat absorbing member, and also allows heat to be appropriately released from the heat radiating portion into the air. As a result, the transfer of heat from the heat absorbing member to the support member, that is, the transfer of heat from the heat absorbing member to the sensor panel via the support member, can be suppressed, thereby suppressing the generation of noise in the sensor panel due to the heat. Therefore, according to the configuration of [1], deterioration in the image quality of a radiation image or the like due to the noise is suppressed, so that the reliability of the radiation imaging device can be improved.
[2] The radiation imaging device according to [1], wherein the heat absorbing member is not adhered to the flexible printed circuit and the IC chip.
   According to the configuration of [2], since the flexible printed circuit and the IC chip are not adhesively fixed to the support member via the heat absorbing member, the flexible printed circuit and the IC chip can be easily replaced when performing repair of the radiation imaging device.
[3] The radiation imaging device according to [1] or [2], wherein the heat absorbing member includes a plurality of sheet members stacked in the first direction.
   According to the configuration of [3], by adjusting the number of sheet members (number of layers) constituting the heat absorbing member, the thickness of the heat absorbing member in the first direction can be easily adjusted so that, for example, the heat absorbing member is pressed against the IC chip with an appropriate force from the viewpoint of effectively transferring heat from the IC chip to the heat absorbing member.
[4] The radiation imaging device according to any one of [1] to [3], wherein the heat absorbing member is disposed outside the detection region when viewed from the first direction.
   According to the configuration of [4], the influence of the heat absorbed by the heat absorbing member on the detection region can be more effectively reduced, so that the generation of noise due to heat in the sensor panel (detection region) can be more effectively suppressed.
[5] The radiation imaging device according to any one of [1] to [4], wherein a thickness of the heat absorbing member in the first direction is substantially uniform over an entire region of the heat absorbing member in the third direction in a natural state, and the heat absorbing portion is in a state of being compressed in the first direction by being sandwiched between the flexible printed circuit and the support member.
   According to the configuration of [5], by configuring such that a compressive force in the first direction is applied to a portion (that is, the heat absorbing portion) disposed between the flexible printed circuit and the support member among members having a substantially uniform thickness in a natural state, the heat absorbing portion and the heat radiating portion that satisfy the magnitude relationship of the thickness defined in [1] can be easily formed.
[6] The radiation imaging device according to any one of [1] to [5], wherein one end of the flexible printed circuit is connected to a terminal provided on a connection surface, which is a surface of the control board opposite to a surface facing the back surface, a capacitor is provided on the flexible printed circuit between the one end and a region where the IC chip is mounted, and the heat absorbing member is disposed so as not to be in contact with the capacitor.
   According to the configuration of [6], it is possible to suppress the transfer of heat generated in the IC chip to the capacitor via the heat absorbing member. As a result, deterioration of the characteristics of the capacitor due to heat can be suppressed.
[7] The radiation imaging device according to any one of [1] to [6], wherein one end of the flexible printed circuit is connected to a terminal provided on a connection surface, which is a surface of the control board opposite to a surface facing the back surface, a capacitor is provided on the flexible printed circuit between the one end and a region where the IC chip is mounted, and an inner end of the contact surface of the heat absorbing portion closest to the control board is located at a position farther from the back surface than the connection surface of the control board in the first direction, whereby the capacitor is disposed so as not to be in contact with the control board.
   In the configuration of [7], by disposing the heat absorbing member (heat absorbing portion) configured as described above between the support member and the IC chip, a portion of the flexible printed circuit between the one end and the region where the IC chip is mounted can be separated from the connection surface of the control board. This can prevent the capacitor from being damaged due to contact with the control board.
[8] The radiation imaging device according to any one of [1] to [7], wherein one end of the flexible printed circuit is connected to a terminal provided on a connection surface, which is a surface of the control board opposite to a surface facing the back surface, and when viewed from the third direction, the contact surface of the heat absorbing portion is inclined so as to become more distant from the back surface along the first direction as it approaches the control board along the second direction.
   According to the configuration of [8], the risk of a portion of the flexible printed circuit near the control board coming into contact with the connection surface of the control board (or the risk of a part of the flexible printed circuit being pressed with a strong force against an edge of the connection surface of the control board) can be reduced, and the total length of the flexible printed circuit can be shortened.
[9] The radiation imaging device according to any one of [1] to [8], wherein the support member includes a base plate having the support surface and the back surface, and a radiation shielding member provided at an edge portion of the base plate when viewed from the first direction, and the heat absorbing member is provided between the radiation shielding member and the IC chip.
   According to the configuration of [9], the radiation shielding member can be interposed between the base plate in the support member, on which the sensor panel is supported, and the heat absorbing member. As a result, the transfer of heat from the heat absorbing member to the base plate, and consequently the transfer of heat to the sensor panel, can be more effectively suppressed.
[10] The radiation imaging device according to [9], wherein a heat capacity of the heat absorbing member is greater than a heat capacity of the radiation shielding member.
   According to the configuration of [10], the transfer of heat from the heat absorbing member to the radiation shielding member can be effectively suppressed, so that the transfer of heat from the heat absorbing member to the sensor panel via the support member (the radiation shielding member and the base plate) can be more effectively suppressed.
[11] The radiation imaging device according to [9] or [10], wherein when viewed from the first direction, the radiation shielding member is disposed at a position overlapping an outer edge portion of the control board or outside the outer edge portion, and a range in which the heat absorbing member is disposed in the second direction is included in a range in which the radiation shielding member is disposed in the second direction.
   According to the configuration of [11], interference (contact) between the heat absorbing member and the control board can be suitably suppressed. This can facilitate assembly work (for example, an operation of connecting one end of the flexible printed circuit to a terminal of the control board) and suppress the transfer of heat generated in the IC chip to the control board via the heat absorbing member.
[12] The radiation imaging device according to any one of [1] to [11], wherein a plurality of the flexible printed circuits are arranged along the third direction, each having the IC chip mounted thereon, and the heat absorbing member extends in the third direction so as to correspond to the plurality of flexible printed circuits.

According to the configuration of [12], by providing one heat absorbing member that is elongated in the third direction for a plurality of IC chips (flexible printed circuits) arranged side by side in the third direction, the volume and heat capacity of the heat absorbing member can be more effectively increased. This can more effectively suppress the transfer of heat from the heat absorbing member to the support member, that is, the transfer of heat from the heat absorbing member to the sensor panel via the support member.

### Advantageous Effects of Invention

According to an aspect of the present disclosure, a radiation imaging device that can improve reliability is provided.

### Brief Description of Drawings

FIG. 1 shows (A) a plan view, (B) a bottom view, and (C) a side view of a radiation imaging device according to an embodiment.
FIG. 2 is a plan view of the internal structure of the housing of the radiation imaging device of FIG. 1.
FIG. 3 is a cross-sectional view taken along line III-III of FIG. 2.
FIG. 4 is an enlarged plan view of a part of the radiation detection panel.
FIG. 5 is a cross-sectional view taken along line V-V of FIG. 4.
FIG. 6 is a diagram illustrating an internal configuration of the light receiving unit and the IC chip.
FIG. 7 is a bottom view of the internal structure of the housing of the radiation imaging device of FIG. 1.
FIG. 8 is a diagram illustrating a positional relationship between the heat absorbing member and the flexible printed circuit.
FIG. 9 is a diagram illustrating a configuration of the heat absorbing portion and the heat radiating portion of the heat absorbing member.

### Description of Embodiments

Hereinafter, an embodiment of the present disclosure will be described in detail with reference to the drawings. In the following description, identical or corresponding elements are denoted by the same reference signs, and redundant description is omitted. In the drawings, some features of the embodiment are exaggerated for clarity. For this reason, the dimensional ratios of each part in the drawings may be different from the actual dimensional ratios.

### [Overall Configuration of Radiation Imaging Device]

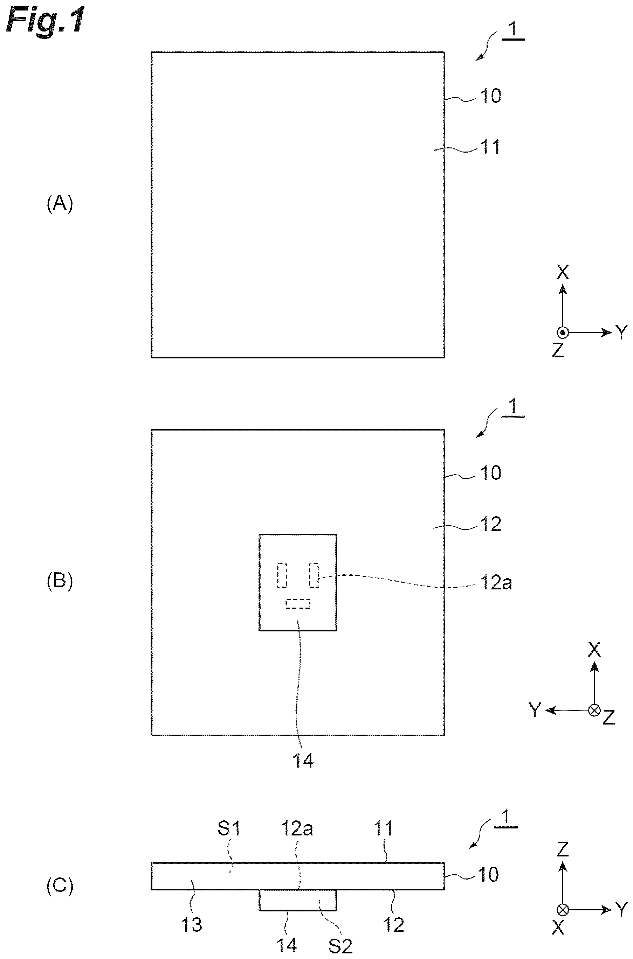

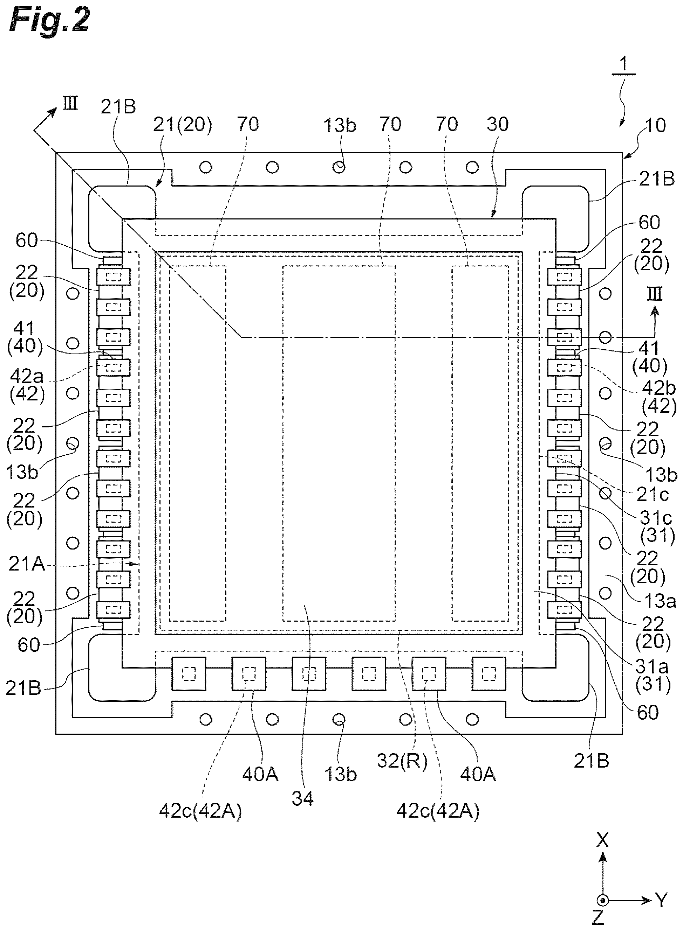

A configuration of a radiation imaging device 1 according to an embodiment will be described with reference to FIGS. 1 to 3. In FIG. 2, a top wall 11 and a fastening member 15, which will be described later, are not illustrated. The radiation imaging device 1 is, for example, a large-area flat panel sensor (X-ray imaging device) used in a medical X-ray imaging system. As illustrated in (A) to (C) of FIG. 1, the radiation imaging device 1 is composed of a substantially rectangular parallelepiped housing 10 and various members housed in the housing 10.

The housing 10 is a substantially rectangular parallelepiped hollow container. The housing 10 has a top wall 11, a bottom wall 12, and a side wall 13. The top wall 11 and the bottom wall 12 are both formed in a rectangular plate shape and are arranged to face each other while being separated from each other. In this specification, a direction in which the top wall 11 and the bottom wall 12 face each other is referred to as a Z-axis direction. Among directions orthogonal to the Z-axis direction, a direction in which a rod-shaped heat absorbing member 60, which will be described later, extends is referred to as an X-axis direction, and a direction orthogonal to the Z-axis direction and the X-axis direction is referred to as a Y-axis direction. The side wall 13 extends along an XZ plane or a YZ plane and is formed in a rectangular annular shape so as to connect an outer edge portion of the top wall 11 and an outer edge portion of the bottom wall 12.

At a substantially central portion of an outer surface of the bottom wall 12, a protruding wall 14 is provided in a box shape so as to form a substantially rectangular parallelepiped space with the outer surface of the bottom wall 12. The bottom wall 12 is provided with one or more (three as an example in this embodiment) insertion holes 12a that allow a space S1, which is surrounded by the top wall 11, the bottom wall 12, and the side wall 13, and a space S2, which is surrounded by the bottom wall 12 and the protruding wall 14, to communicate with each other. For example, in the space S2, a power supply for supplying power to a control board 50, which will be described later, an interface (I/F) circuit for outputting an electrical signal corresponding to radiation detected by a sensor panel 30 (radiation detection panel), which will be described later, to an external PC device (for example, a device for displaying a radiation image), and the like can be disposed. Further, on an outer surface of the protruding wall 14, an external connection terminal (not shown) or the like for connecting to the power supply, circuit, etc. disposed in the space S2 is provided.

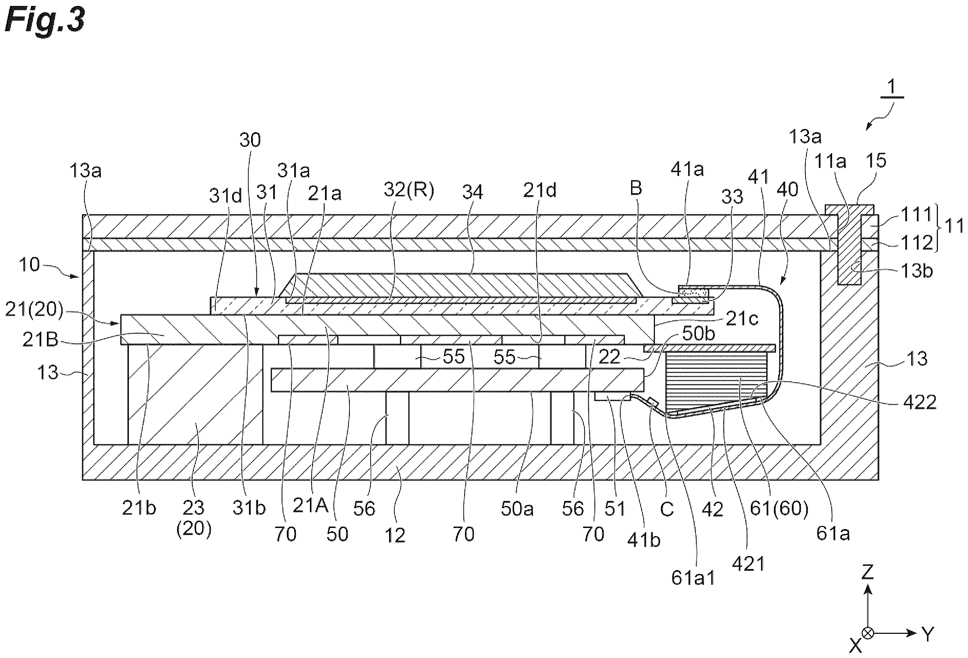

The top wall 11 is formed of a member that transmits radiation (for example, X-rays), which is a detection target of the radiation imaging device 1, to the inside of the housing 10. The top wall 11 guides radiation incident along the Z-axis direction to the inside of the housing 10. That is, the Z-axis direction is the incident direction of the radiation to be detected. In this embodiment, the top wall 11 has a two-layer structure. As illustrated in FIG. 3, the top wall 11 has a carbon fiber plate 111 provided on a side on which radiation is incident (outer side), and a shield member 112 provided on an inner surface of the carbon fiber plate 111 for shielding electromagnetic waves. The shield member 112 is, for example, an aluminum shield formed by adhering aluminum foil to the inner surface of the carbon fiber plate 111.

The bottom wall 12 and the side wall 13 are formed of a metal material (for example, iron or the like) that blocks radiation. As illustrated in FIG. 3, an upper surface 13a of the side wall 13 is in surface contact with the shield member 112 and is electrically connected to the shield member 112. This shields electromagnetic waves from outside the housing 10 toward the inside of the housing 10. As illustrated in FIGS. 2 and 3, a plurality of screw holes 13b are provided on the upper surface 13a of the side wall 13. A fastening member 15 is inserted into a through-hole 11a provided in the top wall 11 and screwed into the screw hole 13b. As a result, the top wall 11 is fixed to the side wall 13.

As illustrated in FIGS. 2 and 3, the radiation imaging device 1 has a support member 20, a sensor panel 30, a plurality of (24 as an example) flexible circuit boards 40, a plurality of (six as an example) flexible circuit boards 40A, a control board 50, a plurality of (four as an example) heat absorbing members 60, and a plurality of (three as an example) radiation shielding substrates 70 housed in the housing 10.

The support member 20 is a member for supporting (fixing) the sensor panel 30, the control board 50, the radiation shielding substrate 70, and the like with respect to the housing 10. In this embodiment, the support member 20 has a base plate 21, a radiation shielding member 22, and a support post 23.

The base plate 21 has a support surface 21a that supports the sensor panel 30 and a back surface 21b opposite to the support surface 21a. The base plate 21 is made of, for example, a metal such as iron, aluminum, stainless steel, a tungsten alloy, or copper tungsten. In this embodiment, as an example, the base plate 21 is formed of relatively lightweight aluminum. The support surface 21a is a surface facing the top wall 11, and the back surface 21b is a surface facing the bottom wall 12. The support surface 21a supports a sensor substrate 31 of the sensor panel 30. The control board 50 is fixed to the back surface 21b via one or more support members 55 formed, for example, in a columnar shape extending in the Z direction.

As illustrated in FIG. 2, the base plate 21 has a main body portion 21A formed in a rectangular shape when viewed from the Z-axis direction (first direction) orthogonal to the support surface 21a, and protruding portions 21B formed at each of the corners (four corners) of the main body portion 21A and protruding outward from the main body portion 21A. In this embodiment, as an example, the main body portion 21A and the protruding portions 21B are integrally formed.

As illustrated in FIGS. 2 and 3, a recessed portion 21d for arranging a plurality of (three in this embodiment) divided radiation shielding substrates 70 is provided on the back surface 21b of the main body portion 21A. Each radiation shielding substrate 70 is formed of, for example, a radiation shielding material such as lead (hard lead) and has the same thickness (for example, about 1 mm). In this embodiment, a rectangular plate-shaped radiation shielding substrate 70 extending in the X-axis direction is provided at each of a central portion and both side edge portions of the main body portion 21A in the Y-axis direction. The radiation shielding substrates 70 are arranged to be separated from each other in the Y-axis direction. That is, a region where the radiation shielding substrate 70 is not provided exists in the recessed portion 21d. As an example, a width (length in the Y-axis direction) of the radiation shielding substrate 70 disposed at the central portion of the main body portion 21A in the Y-axis direction is made larger than a width of the radiation shielding substrates 70 disposed on both sides thereof. Each radiation shielding substrate 70 is disposed in the recessed portion 21d and is adhesively fixed to the back surface 21b of the main body portion 21A (a bottom surface of the recessed portion 21d) via an adhesive member such as a double-sided tape. As illustrated in FIG. 3, a depth (length in the Z-axis direction) of the recessed portion 21d is set such that when the radiation shielding substrate 70 is disposed in the recessed portion 21d, the back surface 21b of a portion of the main body portion 21A where the recessed portion 21d is not provided and a surface of the radiation shielding substrate 70 (a surface facing the control board 50) are substantially flush. That is, the depth of the recessed portion 21d is substantially the same as the thickness (length in the Z-axis direction) of the radiation shielding substrate 70. As described above, by providing the plurality of divided radiation shielding substrates 70 so as to cover only a part of the region of the back surface 21b of the main body portion 21A, the weight of the radiation imaging device 1 can be suppressed as compared with a case where a hard lead plate having an area substantially equal to that of the back surface 21b is provided, while intensively covering a region of the control board 50 where electronic components requiring radiation shielding are disposed. Further, by providing the recessed portion 21d for accommodating the radiation shielding substrate 70 on the back surface 21b of the base plate 21, an increase in the overall size of the radiation imaging device 1 (an increase in length in the Z-axis direction) is suppressed, and the radiation imaging device 1 can be made compact.

The radiation shielding member 22 is formed of a material with high X-ray shielding ability, such as lead or tungsten. In this embodiment, as an example, the radiation shielding member 22 is formed in a strip-like (plate-like) shape and is provided at an edge portion of the back surface 21b of the base plate 21. As illustrated in FIGS. 2 and 3, a part of the radiation shielding member 22 protrudes to the outside of the base plate 21 so as to overlap an IC chip 42 mounted on a flexible printed circuit 41, which will be described later, when viewed from the Z-axis direction. The radiation shielding member 22 may be provided for each IC chip 42, or one radiation shielding member 22 (that is, a member formed in a size that overlaps the plurality of IC chips 42 when viewed from the Z-axis direction) may be provided for a plurality of adjacent IC chips 42. In this embodiment, the latter configuration is adopted. Specifically, at each edge portion of the base plate 21 (main body portion 21A) along the X-axis direction, four radiation shielding members 22 are provided along the X-axis direction. That is, one radiation shielding member 22 is provided so as to overlap three IC chips 42 arranged consecutively in the X-axis direction.

The support post 23 is a member that supports the base plate 21 (protruding portion 21B) with respect to the bottom wall 12. As illustrated in FIG. 3, the support post 23 is provided between the protruding portion 21B and the bottom wall 12 so as to extend in the Z-axis direction. As an example, the support post 23 is formed in a quadrangular prism shape having a size substantially equal to that of the protruding portion 21B when viewed from the Z-axis direction. The support post 23 can be formed of, for example, aluminum, or a metal other than aluminum such as iron, or an engineering plastic such as polyacetal (POM) and polyetheretherketone (PEEK). The support post 23 is fixed to the protruding portion 21B and the bottom wall 12 via, for example, a fixing member (not shown) (for example, a screw or the like). In this embodiment, the base plate 21 is supported with respect to the bottom wall 12 by four support posts 23 provided corresponding to the respective protruding portions 21B provided at the four corners of the main body portion 21A.

The sensor panel 30 has a sensor substrate 31 formed in a rectangular plate shape. The sensor substrate 31 has a first surface 31a on which a light receiving unit 32 (light receiving surface) is formed, and a second surface 31b opposite to the first surface 31a. The first surface 31a is a surface facing the top wall 11, and the second surface 31b is a surface facing the bottom wall 12. A scintillator 34 is disposed on the light receiving unit 32. The scintillator 34 is formed by, for example, depositing a scintillator material containing CsI as a main component on the light receiving unit 32. The scintillator 34 converts radiation incident through the top wall 11 into light. Specifically, the scintillator 34 outputs scintillation light having an intensity corresponding to the incident intensity of radiation to the light receiving unit 32. As a result, a region on the first surface 31a where the light receiving unit 32 is formed functions as a detection region R for detecting radiation. The detection region R has, for example, a light receiving area with a side of about 30 cm to 40 cm (for example, 40 cm x 30 cm).

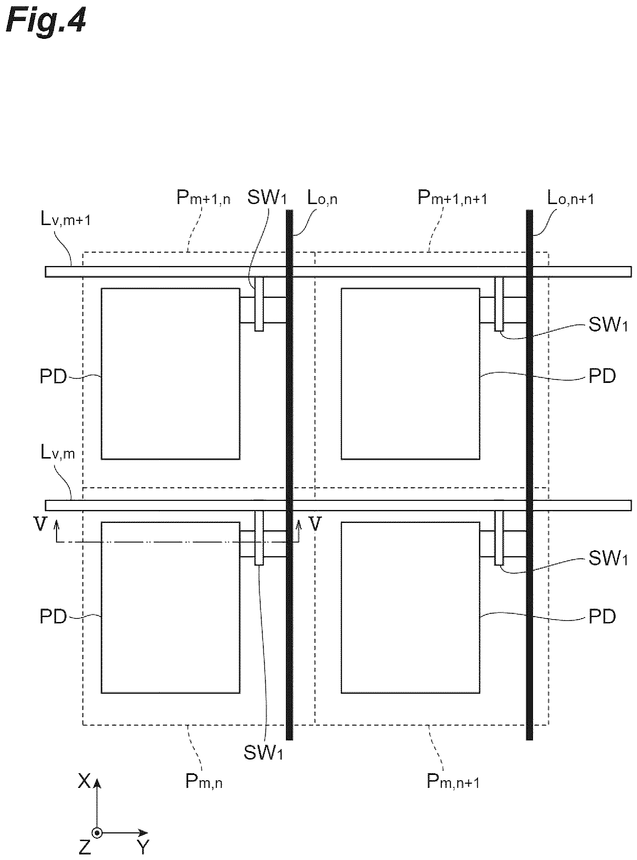

The sensor substrate 31 is, for example, a transparent glass substrate. The sensor substrate 31 is fixed to the base plate 21 by fixing the second surface 31b of the sensor substrate 31 to the support surface 21a of the base plate 21. The second surface 31b is fixed to the support surface 21a via an adhesive member (not shown) such as a double-sided tape. When viewed from the Z direction, at least a region where the light receiving unit 32 and the scintillator 34 are disposed is included in the base plate 21. On the first surface 31a of the sensor substrate 31, a plurality of electrode pads 33 are formed outside the detection region R. The plurality of electrode pads 33 are electrically connected to pixels P_{m,n} (see FIG. 4) formed in the light receiving unit 32 via wirings (readout lines and row selection lines), which will be described later, and the like. In this embodiment, as an example, when viewed from the Z-axis direction, 24 (12 x 2 sides) electrode pads 33 (electrode pads connected to the flexible circuit board 40) are formed on the left and right edge portions of the sensor substrate 31 along the X-axis direction. Further, six electrode pads 33 (electrode pads connected to the flexible circuit board 40A) are formed on one edge portion of the sensor substrate 31 along the Y-axis direction (in this embodiment, the lower edge portion in FIG. 2).

The flexible circuit board 40 is a circuit member electrically connected to the electrode pad 33. The flexible circuit board 40 has a flexible printed circuit 41 that can be deformed, such as by bending, and an IC chip 42 mounted on the flexible printed circuit 41. As illustrated in FIG. 2, in this embodiment, when viewed from the Z-axis direction, a plurality of (12 for each side in this embodiment) flexible circuit boards 40 are arranged at equal intervals along the left and right side portions of the sensor substrate 31 along the X-axis direction. Each flexible circuit board 40 is connected to a corresponding electrode pad 33.

As illustrated in FIG. 3, the flexible printed circuit 41 is arranged to connect the sensor panel 30 (electrode pad 33) and the control board 50 (connector 51) through a side of the base plate 21 when viewed from the Z-axis direction (that is, bent so as not to interfere with the base plate 21 and the radiation shielding member 22). The flexible printed circuit 41 has, for example, a structure in which a circuit pattern of a conductor foil (for example, copper or the like) is formed on an insulator (for example, polyimide or the like) on a thin film. One end portion 41a of the flexible printed circuit 41 is connected to the electrode pad 33 via a connection member B. The connection member B is a member that generates adhesive force by thermocompression bonding, and is, for example, an anisotropic conductive material such as an ACF (Anisotropic Conductive Film) or an ACP (Anisotropic Conductive Paste). The other end portion 41b (one end) of the flexible printed circuit 41 is connected to a connector 51 (terminal) of the control board 50.

The IC chip 42 is a readout circuit (ROIC: Readout IC) that executes processing for reading out a signal from the sensor panel 30. An operation example of the IC chip 42 will be described later.

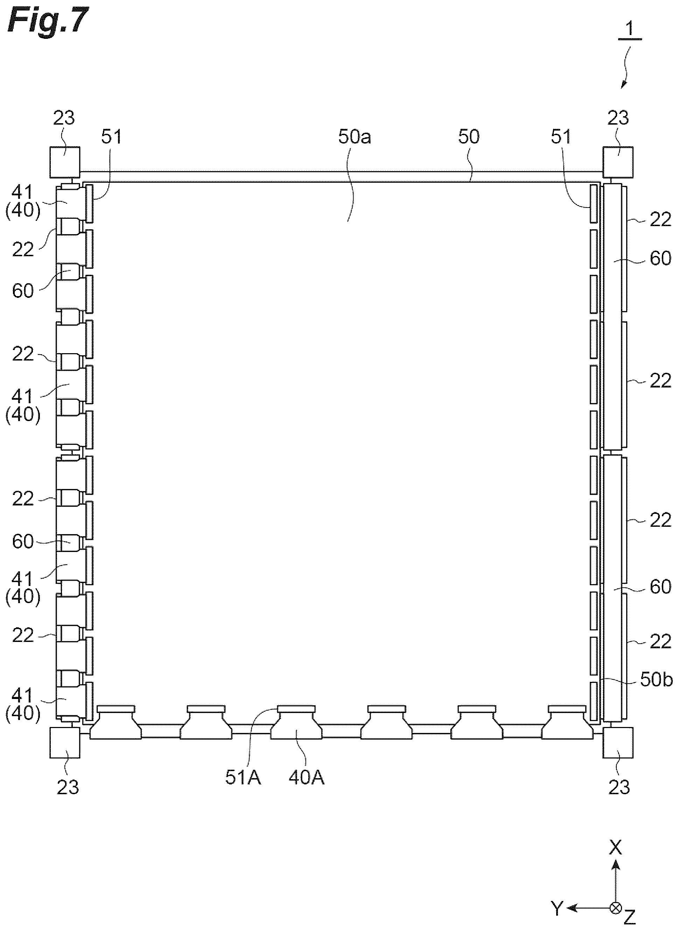

A plurality of (six in this embodiment) flexible circuit boards 40A are arranged at equal intervals on a side portion (lower side in FIG. 2) of the sensor substrate 31 along the Y-axis direction. Each flexible circuit board 40A is connected to a corresponding electrode pad 33. The flexible circuit board 40A is formed in a different shape and size from the flexible circuit board 40, but its basic structure is the same as that of the flexible circuit board 40. An IC chip 42A is mounted on each flexible circuit board 40A. As illustrated in FIG. 3, an end portion on the control board 50 side of each flexible circuit board 40A is connected to a connector 51A (see FIG. 7) provided along a side portion (lower side in FIG. 2) of the control board 50 along the Y-axis direction.

The control board 50 is a circuit board that is disposed at a position facing the back surface 21b of the base plate 21 and processes a signal read out from the sensor panel 30. The control board 50 has a connection surface 50a provided with the connector 51 to be connected to the end portion 41b of the flexible printed circuit 41 described above. The connection surface 50a is a surface facing the bottom wall 12, and is a surface opposite to a surface of the control board 50 facing the base plate 21 (an upper surface of the control board 50 in FIG. 3).

The control board 50 includes a circuit or the like for controlling the operation of the IC chip 42 and supplying power to the IC chip 42. In this embodiment, the control board 50 disposed in the space S1 (see (C) of FIG. 1) is electrically connected to the above-described power supply, I/F circuit, and the like disposed in the space S2 (see (C) of FIG. 1) via a conductive member (for example, wiring, lead pins, or the like) inserted into the insertion hole 12a. The control board 50 is fixed to the back surface 21b of the base plate 21 via the one or more support members 55 described above. In this embodiment, in order to improve the support stability of the base plate 21, a plurality of support members 56 are provided for supporting the base plate 21 with respect to the bottom wall 12 by being inserted into through-holes (not shown) provided in the control board 50. The control board 50 is not supported by the support members 56, but is fixed to the base plate 21 by the support members 55 as described above.

### [Operation of Radiation Imaging Device]

Next, an example of the operation (radiation detection) of the radiation imaging device 1 will be described. In this embodiment, an amplifier chip for signal reading (signal connection portion) is formed on the IC chip 42 (ROIC) of the flexible circuit board 40 connected to the electrode pads 33 formed on a peripheral portion of the sensor substrate 31 along the X-axis direction. As an example, a signal connection portion 42a is formed by the IC chip 42 of the flexible circuit board 40 provided on the left side of the sensor substrate 31 in FIG. 2, and a signal connection portion 42b is formed by the IC chip 42 of the flexible circuit board 40 provided on the right side of the sensor substrate 31. As described above, in this embodiment, a configuration in which a signal readout line (data line) is divided into upper and lower parts is adopted in order to reduce noise and improve the speed of signal readout. However, it is not essential to divide the signal readout line in this way. The same applies to other data lines related to signal control. On the other hand, a vertical shift register (vertical scanning circuit) 42c is formed on the IC chip 42A of the flexible circuit board 40A connected to the electrode pads 33 formed on the edge portion (lower side in FIG. 2) of the sensor substrate 31 along the Y-axis direction.

An example of the operation of the radiation imaging device 1 will be described with reference to FIGS. 4 to 6. As illustrated in FIG. 4, the light receiving unit 32 is configured with M x N pixels arranged two-dimensionally in M rows and N columns. A pixel P_{m,n} is a pixel located in the m-th row and the n-th column. Here, m is each integer from 1 to M, and n is each integer from 1 to N. In FIG. 4, the column direction coincides with the X-axis direction, and the row direction coincides with the Y-axis direction. Each of the plurality of pixels P_{1,1} to P_{M,N} included in the light receiving unit 32 includes a photodiode PD and a readout switch SW1. A bias voltage is applied to an anode terminal of the photodiode PD, and one end (one current terminal) of the readout switch SW1 is connected to a cathode terminal of the photodiode PD. The other end (the other current terminal) of the readout switch SW1 is connected to a corresponding readout line (for example, in the case of the pixel P_{m,n}, the n-th column readout line L_{O,n}). A control terminal of the readout switch SW1 is connected to a corresponding row selection line (for example, in the case of the pixel P_{m,n}, the m-th row selection line L_{V,m}).

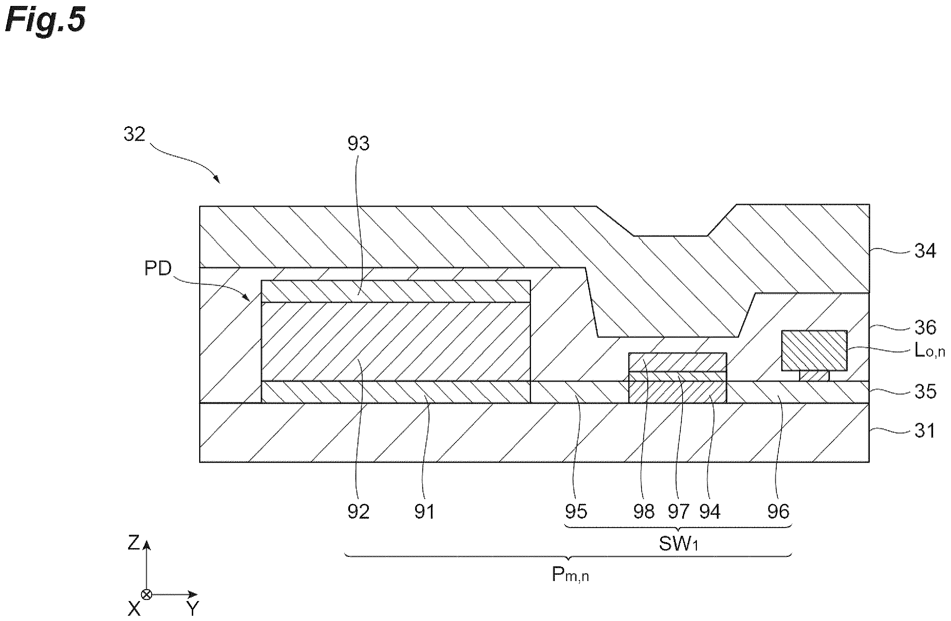

As illustrated in FIG. 5, a silicon film 35 is provided on the entire first surface 31a of the sensor substrate 31. The photodiode PD, the readout switch SW1, and the n-th column readout line L_{O,n} are formed on the surface of this silicon film 35. The photodiode PD, the readout switch SW1, and the n-th column readout line L_{O,n} are covered by an insulating layer 36. On the insulating layer 36, the scintillator 34 is provided so as to cover the entire detection region R of the first surface 31a of the sensor substrate 31. The photodiode PD includes, for example, amorphous silicon.

The photodiode PD of this embodiment has an n-type semiconductor layer 91 made of n-type polycrystalline silicon, an i-type semiconductor layer 92 made of i-type amorphous silicon provided on the n-type semiconductor layer 91, and a p-type semiconductor layer 93 made of p-type amorphous silicon provided on the i-type semiconductor layer 92. The readout switch SW1 is a thin film transistor (TFT) formed of polycrystalline silicon and has a configuration as a field effect transistor (FET). That is, the readout switch SW1 has a channel region 94, a source region 95 disposed along one side surface of the channel region 94, a drain region 96 disposed along the other side surface of the channel region 94, and a gate insulating film 97 and a gate electrode 98 formed on the channel region 94. The n-th column readout line L_{O,n} is made of metal. The scintillator 34 generates scintillation light according to the incident radiation, converts a radiation image into an optical image, and outputs this optical image to the light receiving unit 32.

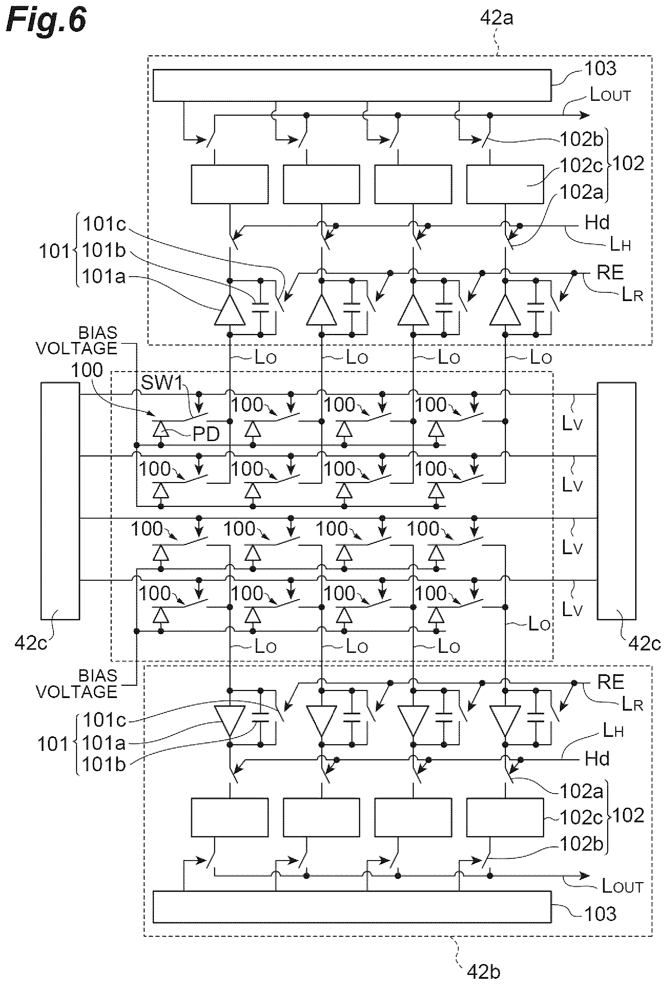

FIG. 6 shows 4x4 pixels 100 as representatives of M x N pixels P_{m,n} (m=1,...,M, n=1,...,N). Each of the pixels 100 is configured to include a photodiode PD and a readout switch SW1. The photodiode PD generates an amount of charge corresponding to the intensity of incident light and accumulates the generated charge in a junction capacitance portion. As described above, the readout switch SW1 is connected to a row selection line L_{V} corresponding to a row to which the pixel 100 belongs. Here, the row selection line L_{V} corresponding to the pixel P_{m,n} in the m-th row is the m-th row selection line L_{V,m} described above. The M row selection lines L_{V} are connected to a vertical shift register 42c. Each of the vertical shift registers 42c generates a row selection signal for controlling a conduction state/non-conduction state of the readout switch SW1 for each row, and sequentially provides the row selection signal to the row selection line L_{V} of each row.

When a row selection signal output from the vertical shift register 42c to the row selection line L_{V} is at a non-significant value (for example, a low level), the readout switch SW1 opens. At this time, the charge generated in the photodiode PD is accumulated in the junction capacitance portion without being output to the corresponding column readout line L_{O}. Here, the column readout line L_{O} corresponding to the pixel P_{m,n} in the n-th column is the n-th column readout line L_{O,n} described above. On the other hand, when the row selection signal is at a significant value (for example, a high level), the readout switch SW1 closes. At this time, the charge that has been generated in the photodiode PD and accumulated in the junction capacitance portion until then is output to the corresponding readout line L_{O} via the readout switch SW1. The output charge is sent to an integration circuit 101 via the readout line L_{O}. In this embodiment, among the pixels 100 formed in the light receiving unit 32, the readout switch SW1 of the pixel 100 located in a row on the upper side of the sensor substrate 31 is connected to the integration circuit 101 of the signal connection portion 42a via the corresponding readout line L_{O}. On the other hand, among the pixels 100 formed in the light receiving unit 32, the readout switch SW1 of the pixel 100 located in a row on the lower side of the sensor substrate 31 is connected to the integration circuit 101 of the signal connection portion 42b via the corresponding readout line L_{O}. The method of dividing the rows on the upper side and the lower side of the sensor substrate 31 is arbitrary. For example, when the number of rows on the upper side of the sensor substrate 31 is N1 and the number of rows on the lower side of the sensor substrate 31 is N2, any of the relationships "N1=N2", "N1>N2", and "N1<N2" may be satisfied.

The integration circuit 101 has a so-called charge integration type configuration including an amplifier 101a, a capacitive element 101b, and a discharge switch 101c. The capacitive element 101b and the discharge switch 101c are connected in parallel with each other, and are connected between an input terminal and an output terminal of the amplifier 101a. The input terminal of the amplifier 101a is connected to the column readout line L_{O}. A reset control signal RE is provided to the discharge switch 101c via a reset line L_{R}.

The reset control signal RE instructs an opening/closing operation of the discharge switch 101c of each of the N integration circuits 101. For example, when the reset control signal RE is at a non-significant value (for example, a high level), the discharge switch 101c closes, the capacitive element 101b is discharged, and an output voltage value of the integration circuit 101 is initialized. When the reset control signal RE is at a significant value (for example, a low level), the discharge switch 101c opens, the charge input to the integration circuit 101 is accumulated in the capacitive element 101b, and a voltage value corresponding to the accumulated charge amount is output from the integration circuit 101.

Each of the signal connection portions 42a and 42b further has N holding circuits 102 and a horizontal shift register 103. Each holding circuit 102 includes an input switch 102a, an output switch 102b, and a voltage holding unit 102c. One end of the voltage holding unit 102c is connected to an output end of the integration circuit 101 via the input switch 102a, and the other end of the voltage holding unit 102c is connected to a voltage output line L_{OUT} via the output switch 102b. A holding control signal Hd is given to the input switch 102a via a holding line L_{H}. The holding control signal Hd instructs an opening/closing operation of the input switch 102a of each of the N holding circuits 102. A column selection signal is given from the horizontal shift register 103 to the output switch 102b of the holding circuit 102. The column selection signal instructs an opening/closing operation of the output switch 102b of the holding circuit 102 of the corresponding column.

When the holding control signal Hd changes from a high level to a low level, the input switch 102a changes from a closed state to an open state, and the voltage value input to the holding circuit 102 at that time is held in the voltage holding unit 102c. Thereafter, when the column selection signal from the horizontal shift register 103 sequentially changes from a low level to a high level for each column, the output switch 102b sequentially closes, and the voltage value held in the voltage holding unit 102c is sequentially output to the voltage output line L_{OUT} for each column.

### [Configuration of Heat Absorbing Member]

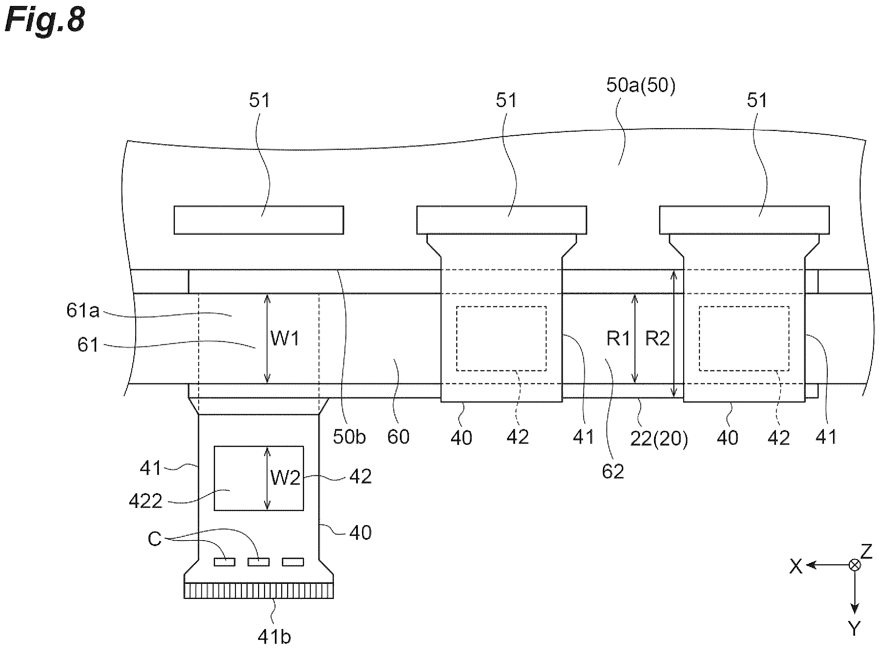

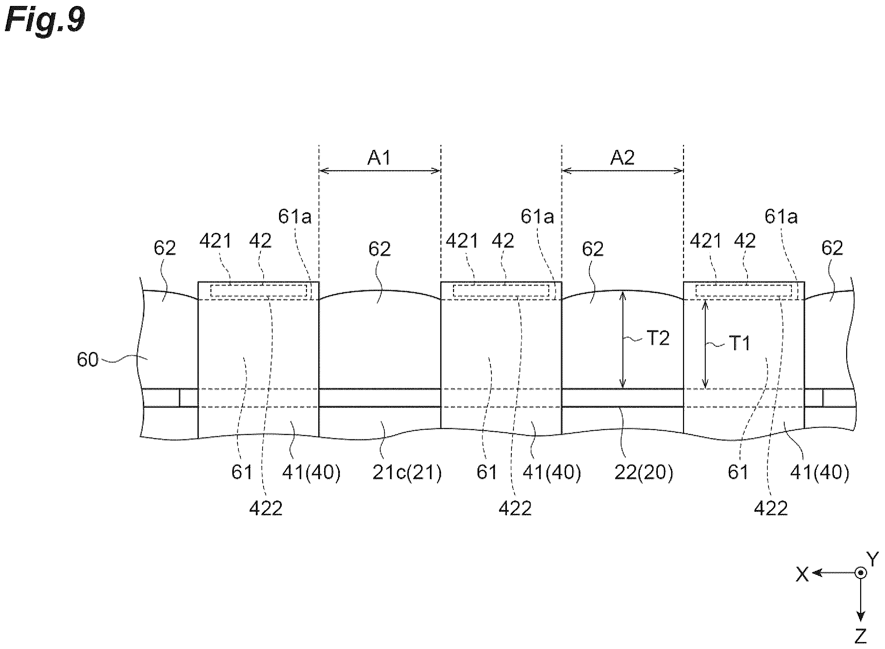

Next, the configuration of the heat absorbing member 60 will be described in more detail mainly with reference to FIGS. 3 and 7 to 9. FIG. 7 is a view of the structure inside the housing 10 as seen from a side facing a lower surface (connection surface 50a) of the control board 50. In FIG. 7, illustration of a plurality of flexible circuit boards 40 arranged along the X-axis direction at one edge portion (right side in FIG. 7) of the control board 50 in the Y-axis direction is omitted. That is, the right side portion of FIG. 7 shows a state before the flexible circuit board 40 is attached. For the leftmost flexible printed circuit 41 illustrated in FIG. 8, a state is shown in which an end portion 41b is detached from the connector 51 and extends outward in the Y-axis direction. FIG. 9 is a view of the heat absorbing member 60 disposed between the support member 20 (radiation shielding member 22) and the IC chip 42 mounted on each flexible printed circuit 41, as seen from the side (Y-axis direction).

The heat absorbing member 60 is provided between the support member 20 and the IC chip 42 on a side opposite to a side where the sensor panel 30 is located with respect to the support member 20 (the base plate 21 and the radiation shielding member 22), and absorbs heat from the IC chip 42. The heat absorbing member 60 is formed in a rod shape extending in the X-axis direction. The heat absorbing member 60 is constituted by a plurality of sheet members stacked in the Z-axis direction. As an example, the heat absorbing member 60 is formed by stacking seven sheet members each having a thickness of 1 mm and pressing or welding them together. The material constituting the heat absorbing member 60 is, for example, acrylic, silicone, ceramic, or the like.

In this embodiment, the heat absorbing member 60 is provided between the IC chip 42 mounted on each flexible printed circuit 41 and the radiation shielding member 22. In the example of FIG. 3, the heat absorbing member 60 is disposed so as to overlap a portion of the radiation shielding member 22 that protrudes outward from an end portion 21c of the base plate 21 in the Y-axis direction. That is, in the Z-axis direction, the heat absorbing member 60 does not overlap the base plate 21. However, the arrangement configuration of the heat absorbing member 60 is not limited to the above configuration. For example, at least a part of the heat absorbing member 60 may be disposed so as to overlap both the radiation shielding member 22 and the base plate 21. Further, for example, in a case where the base plate 21 itself is formed of a radiation shielding material and the radiation shielding member 22 is omitted, the heat absorbing member 60 may be provided between the base plate 21 and the IC chip 42.

As illustrated in FIG. 7, the radiation imaging device 1 includes a plurality of (12 in this embodiment) flexible printed circuits 41 arranged along the X-axis direction (third direction) at each of both side edge portions of the control board 50 in the Y-axis direction. Further, at each edge portion, two rod-shaped heat absorbing members 60 are arranged side by side in the X-axis direction. That is, in this embodiment, one heat absorbing member 60 has a length of approximately half the length of the control board 50 in the X-axis direction so as to correspond to a plurality of (six in this embodiment) flexible printed circuits 41 (in other words, so as to have a portion disposed between a plurality of IC chips 42 on the plurality of flexible printed circuits 41 and the radiation shielding member 22). As described above, in this embodiment, one radiation shielding member 22 is provided for three flexible printed circuits 41. Therefore, as shown in the right side portion of FIG. 7, one heat absorbing member 60 is provided so as to straddle two radiation shielding members 22 arranged side by side in the X-axis direction, and is sandwiched between the IC chips 42 on the six flexible printed circuits 41 and the two radiation shielding members 22. The heat absorbing member 60 may be fixed to the radiation shielding member 22 by a double-sided tape or the like, or may not be fixed. Even when the heat absorbing member 60 is not fixed, the heat absorbing member 60 is sandwiched and fixed by the flexible printed circuit 41 and the radiation shielding member 22 as will be described later. According to the configuration in which the heat absorbing member 60 is fixed to the radiation shielding member 22, displacement or the like of the heat absorbing member 60 can be suppressed. On the other hand, according to the configuration in which the heat absorbing member 60 is not fixed to the radiation shielding member 22, replacement work of the heat absorbing member 60, the radiation shielding member 22, and the like becomes easy.

As illustrated in FIGS. 8 and 9, the heat absorbing member 60 has a heat absorbing portion 61 and a heat radiating portion 62.

The heat absorbing portion 61 is a portion disposed in a region overlapping the flexible printed circuit 41 when viewed from the Z-axis direction (that is, a portion sandwiched between the flexible printed circuit 41 and the radiation shielding member 22). The heat absorbing portion 61 has a contact surface 61a that contacts a surface 422 of the IC chip 42 opposite to a mounting surface 421 of the IC chip 42 on the flexible printed circuit 41. As illustrated in FIG. 8, as an example, the IC chip 42 is disposed at a central portion in a width direction (X-axis direction) of the flexible printed circuit 41 and has a rectangular plate shape. A width W1 of the contact surface 61a along the Y-axis direction is set to be equal to or greater than a width W2 of the IC chip 42 along the Y-axis direction. As a result, the entire surface 422 of the IC chip 42 is configured to be in contact with the contact surface 61a.

The heat radiating portion 62 is a portion that is connected to the heat absorbing portion 61 and extends outward beyond an outer edge of the flexible printed circuit 41 in the X-axis direction (third direction) intersecting the extending direction (second direction, Y-axis direction) of the flexible printed circuit 41 when viewed from the Z-axis direction. In this embodiment, as described above, since the heat absorbing member 60 is formed in a rod shape extending in the X-axis direction, the heat radiating portion 62 is integrally (continuously) formed of the same member (material) as the heat absorbing portion 61. In a case where one heat absorbing member 60 is formed so as to straddle a plurality of flexible printed circuits 41 as in this embodiment, the heat radiating portion 62 disposed between two heat absorbing portions 61 located on adjacent flexible printed circuits 41 in the X-axis direction is shared by the two heat absorbing portions 61. For example, when focusing on the heat absorbing portion 61 provided corresponding to the flexible printed circuit 41 at the central portion in FIG. 9, the heat radiating portion 62 corresponding to the heat absorbing portion 61 is a portion included in regions A1 and A2 on both sides of the heat absorbing portion 61 in the heat absorbing member 60. In this case, the heat radiating portion 62 corresponding to the region A1 is shared by the heat absorbing portion 61 provided corresponding to the flexible printed circuit 41 at the central portion in FIG. 9 and the heat absorbing portion 61 provided corresponding to the flexible printed circuit 41 at the left end in FIG. 9. Similarly, the heat radiating portion 62 corresponding to the region A2 is shared by the heat absorbing portion 61 provided corresponding to the flexible printed circuit 41 at the central portion in FIG. 9 and the heat absorbing portion 61 provided corresponding to the flexible printed circuit 41 at the right end in FIG. 9.

As illustrated in FIG. 9, a thickness T2 of the heat radiating portion 62 in the Z-axis direction is greater than a thickness T1 of the heat absorbing portion 61 in the Z-axis direction. As illustrated in FIG. 9, the thickness T2 of the heat radiating portion 62 varies depending on the position in the X-axis direction. For example, when the heat absorbing portion 61 is compressed in the Z-axis direction by the flexible printed circuit 41 and the radiation shielding member 22 as in this embodiment, the thickness T2 tends to increase as the portion of the heat radiating portion 62 is farther from the heat absorbing portion 61 in the **X-axis** direction. Therefore, "the thickness T2 of the heat radiating portion 62" when determining whether the relationship "T2 > T1" between the thicknesses T1 and T2 described above is satisfied means an average value of the thickness of the entire region of the heat radiating portion 62 in the X-axis direction.

Further, as illustrated in FIG. 3, the thickness T1 of the heat absorbing portion 61 may vary depending on the position in the Y-axis direction. As a result, the thickness T2 of the heat radiating portion 62 adjacent to the heat absorbing portion 61 may also vary depending on the position in the Y-axis direction. In such a case, "the thickness T2 of the heat radiating portion 62 in the Z-axis direction is greater than the thickness T1 of the heat absorbing portion 61 in the Z-axis direction" means that "T2 > T1" is satisfied at any position in the Y-axis direction. Although it is considered that the thickness T1 of the heat absorbing portion 61 does not change significantly depending on the position in the X-axis direction because it is pressed by the flexible printed circuit 41, if the thickness T1 changes significantly depending on the position in the X-axis direction, "the thickness T1 of the heat absorbing portion 61" when determining whether the relationship "T2 > T1" between the thicknesses T1 and T2 described above is satisfied means an average value of the thickness of the entire region of the heat absorbing portion 61 in the X-axis direction.

From the viewpoint of sufficiently securing the thickness T2 of the heat radiating portion 62 and improving the heat radiating property of the heat radiating portion 62, the thickness T2 of the heat radiating portion 62 is preferably larger than an average value of the thickness of **the entire** region of the heat absorbing portion 61 (that is, a portion of the heat absorbing member 60 overlapping the flexible printed circuit 41 in the Z-axis direction), more preferably larger than an average value of the thickness of a portion of the heat absorbing portion 61 excluding a region overlapping the IC chip 42 (that is, a region that can be deformed to have a smaller thickness than other portions by being pressed against the IC chip 42), and still more preferably larger than the thickness of the thickest portion of the heat absorbing portion 61.

### [Effects]

In the radiation imaging device 1 described above, the heat absorbing member 60 has the heat absorbing portion 61, which is disposed so as to overlap the flexible printed circuit 41 and efficiently absorbs heat from the IC chip 42 via the contact surface 61a, and the heat radiating portion 62, which is formed to extend outward in the X-axis direction from the heat absorbing portion 61. By providing the heat radiating portion 62 in addition to the heat absorbing portion 61, and by providing the heat radiating portion 62, which is thicker than the heat absorbing portion 61, at a position that does not overlap the flexible printed circuit 41 in the Z-axis direction, the overall volume and heat capacity of the heat absorbing member 60 can be effectively increased, and the surface area of the portion of the heat radiating portion 62 that is in contact with air can be appropriately secured. This allows heat from the IC chip 42 to be effectively absorbed by the heat absorbing member 60, and also allows heat to be appropriately released from the heat radiating portion 62 into the air. That is, heat can be efficiently released through a path of "IC chip 42 -> heat absorbing portion 61 -> heat radiating portion 62 -> air (space S1 in the housing 10)". As a result, the transfer of heat from the heat absorbing member 60 to the support member 20, that is, the transfer of heat from the heat absorbing member 60 to the sensor panel 30 via the support member 20 (in this embodiment, the radiation shielding member 22 and the base plate 21), can be suppressed, thereby suppressing the generation of noise in the sensor panel 30 (light receiving unit 32) due to the heat. Therefore, according to the radiation imaging device 1, deterioration in the image quality of a radiation image or the like due to the noise is suppressed, so that reliability can be improved.

Further, the heat absorbing member 60 itself does not have adhesiveness, and is not adhered to at least the flexible printed circuit 41 and the IC chip 42. That is, the contact surface 61a of the heat absorbing portion 61 is not adhesively fixed to the surface 422 or the like of the IC chip 42. Therefore, since the flexible printed circuit 41 and the IC chip 42 are not adhesively fixed to the support member 20 via the heat absorbing member 60, the flexible printed circuit 41 and the IC chip 42 can be easily replaced when performing repair of the radiation imaging device 1.

Further, the heat absorbing member 60 is configured by a plurality of (seven in this embodiment) sheet members stacked in the Z-axis direction. According to this configuration, by adjusting the number of sheet members (number of layers) constituting the heat absorbing member 60, the thickness of the heat absorbing member 60 in the Z-axis direction can be easily adjusted so that, for example, the heat absorbing member 60 (the contact surface 61a of the heat absorbing portion 61) is pressed against the surface 422 of the IC chip 42 with an appropriate force from the viewpoint of effectively transferring heat from the IC chip 42 to the heat absorbing member 60. Further, according to this configuration, since a side surface (a side surface intersecting the X-axis direction or the Y-axis direction) of the heat absorbing member 60 does not become a completely flat surface (for example, some sheet members slightly protrude in the X-axis direction or the Y-axis direction more than adjacent sheet members), a contact area between the heat absorbing member 60 and air increases, and an effect of improving heat radiation is also obtained.

Further, as illustrated in FIG. 3, when viewed from the Z-axis direction, the heat absorbing member 60 is disposed outside the detection region R (effective light-receiving area). That is, the heat absorbing member 60 that absorbs heat from the IC chip 42 is separated from the detection region R to an extent that they do not overlap at least in the Z-axis direction. According to this configuration, the influence of the heat absorbed by the heat absorbing member 60 on the detection region R can be more effectively reduced, so that the generation of noise due to heat in the sensor panel 30 (light receiving unit 32) can be more effectively suppressed.

Further, a thickness of the heat absorbing member 60 in the Z-axis direction is substantially uniform over an entire region of the heat absorbing member 60 in the X-axis direction in a natural state. Here, the "natural state" means a state in which the heat absorbing member 60 is not sandwiched between the flexible printed circuit 41 (IC chip 42) and the support member 20 (radiation shielding member 22), and a force for compressing in the Z-axis direction is not acting on the heat absorbing member 60. Further, as illustrated in FIGS. 3 and 9, the heat absorbing portion 61 is in a state of being compressed in the Z-axis direction by being sandwiched between the flexible printed circuit 41 (IC chip 42) and the support member 20 (radiation shielding member 22). That is, the relationship "T2 > T1" between the thickness T2 of the heat radiating portion 62 and the thickness T1 of the heat absorbing portion 61 described above is realized by a force for compressing in the Z-axis direction acting on the heat absorbing portion 61. For example, by adjusting a length from an end portion 41a to an end portion 41b of the flexible printed circuit 41, a position where the end portion 41a or the end portion 41b is connected to a facing member (an electrode pad 33, a connector 51, or the like), a thickness of the heat absorbing member 60 (for example, a thickness, a number of sheets, or the like of sheet members constituting the heat absorbing member 60), and the like, it can be configured such that the heat absorbing portion 61 is compressed in the Z-axis direction as described above. That is, by configuring such that a force for compressing in the Z-axis direction is applied to a portion (that is, the heat absorbing portion 61) disposed between the flexible printed circuit 41 and the support member 20 among the heat absorbing members 60 having a substantially uniform thickness in a natural state, the heat absorbing portion 61 and the heat radiating portion 62 can be easily formed so as to satisfy the magnitude relationship "T2 > T1" of the thicknesses T1 and T2 described above. Further, by applying pressure to the heat absorbing portion 61 in this way and bringing the IC chip 42 and the heat absorbing portion 61 into contact with each other, for example, when an impact is applied to the heat absorbing member 60 from the outside, the contact between the heat absorbing portion 61 and the IC chip 42 is less likely to be released. Further, by pressing the heat absorbing portion 61 against the surface 422 of the IC chip 42, the heat absorbing portion 61 can be pushed into a space facing a side surface of the IC chip 42, so that the efficiency of heat absorption from the IC chip 42 by the heat absorbing portion 61 can be improved.

Further, as illustrated in FIGS. 3 and 8, a capacitor C (a plurality of capacitors C in this embodiment) is provided on the flexible printed circuit 41 between an end portion 41b connected to the connector 51 of the control board 50 and a region where the IC chip 42 is mounted. As illustrated in FIG. 3, the heat absorbing member 60 (heat absorbing portion 61) is disposed so as not to be in contact with the capacitor C. That is, a width of the heat absorbing portion 61 in the Y-axis direction is set such that the heat absorbing portion 61 overlaps the surface 422 of the IC chip 42 but does not overlap the capacitor C. According to this configuration, it is possible to suppress the transfer of heat generated in the IC chip 42 to the capacitor C via the heat absorbing member 60 (heat absorbing portion 61). As a result, deterioration of the characteristics of the capacitor C due to heat can be suppressed. Further, it is also possible to suppress the occurrence of damage or peeling of the capacitor C due to the heat absorbing member 60 coming into contact with the capacitor C.

Further, as illustrated in FIG. 3, an inner end portion 61a1 of the contact surface 61a of the heat absorbing portion 61 closest to the control board 50 is located at a position farther from the back surface 21b than the connection surface 50a of the control board 50 in the Z-axis direction. That is, when viewed from the X-axis direction, the inner end portion 61a1 of the contact surface 61a located closest to the control board 50 in the Y-axis direction is located on the bottom wall 12 side with respect to the connection surface 50a. As a result, the capacitor C is disposed so as not to be in contact with the control board 50 (connection surface 50a). That is, by disposing the heat absorbing member 60 (heat absorbing portion 61) configured as described above between the support member 20 (radiation shielding member 22) and the IC chip 42, a portion of the flexible printed circuit 41 between the end portion 41b and the region where the IC chip 42 is mounted (that is, a portion where the capacitor C is disposed) can be separated from the connection surface 50a of the control board 50. This can prevent the capacitor C from being damaged by contacting the control board 50. Furthermore, for example, consider a case where the end portion 41b of the flexible printed circuit 41 is connected to the connector 51 in a state where the control board 50 is located above the base plate 21 (that is, a state where the heat absorbing member 60 can be placed on the radiation shielding member 22). In this case, since the inner end portion 61a1 is disposed above the connection surface 50a, the portion of the flexible printed circuit 41 where the capacitor C is disposed is in a state of floating above the connection surface 50a. Therefore, according to this configuration, contact between the capacitor C and the control board 50 can be appropriately prevented even during manufacturing of the radiation imaging device 1 (that is, when connecting the end portion 41b of the flexible printed circuit 41 to the connector 51).

Further, as illustrated in FIG. 3, when viewed from the X-axis direction, the contact surface 61a of the heat absorbing portion 61 is inclined so as to become more distant from the back surface 21b along the Z-axis direction (that is, to approach the bottom wall 12) as it approaches the control board 50 along the Y-axis direction. The configuration in which the contact surface 61a is inclined in this way is formed, for example, for the following reason. When the heat absorbing portion 61 is pressed by the flexible printed circuit 41 and the radiation shielding member 22, a pushing force toward the radiation shielding member 22 side acts more strongly on an outermost portion (a portion farthest from the control board 50) of the contact surface 61a of the heat absorbing portion 61. As a result, the inclined shape as described above is formed. According to this configuration, the risk of a portion of the flexible printed circuit 41 near the control board 50 (inner end portion 61a1) coming into contact with the connection surface 50a of the control board 50 (or the risk of a part of the flexible printed circuit 41 being pressed with a strong force against an edge of the connection surface 50a of the control board 50) can be reduced. Further, in order to avoid the contact between the capacitor C and the control board 50 as described above, in a case where the inner end portion 61a1 is configured to be located on the bottom wall 12 side with respect to the connection surface 50a, the total length of the flexible printed circuit 41 can be shortened by inclining the contact surface 61a as shown in FIG. 3.

Further, in this embodiment, the heat absorbing member 60 is provided between the radiation shielding member 22 and the IC chip 42. According to this configuration, the radiation shielding member 22 can be interposed between the base plate 21 on which the sensor panel 30 is supported and the heat absorbing member 60. As a result, heat transfer from the heat absorbing member 60 to the base plate 21, and consequently heat transfer to the sensor panel 30 (light receiving unit 32), can be more effectively suppressed.

Further, in a case where the heat absorbing member 60 is provided so as to be in contact with the radiation shielding member 22 as described above, it is preferable that a heat capacity of the heat absorbing member 60 is larger than a heat capacity of the radiation shielding member 22. According to this configuration, even when the radiation shielding member 22 is formed of a material with excellent thermal conductivity such as copper tungsten, by providing such a heat capacity difference, the transfer of heat from the heat absorbing member 60 to the radiation shielding member 22 can be effectively suppressed. This can more effectively suppress the transfer of heat from the heat absorbing member 60 to the sensor panel 30 via the support member 20 (the radiation shielding member 22 and the base plate 21).

Further, when viewed from the Z-axis direction, the radiation shielding member 22 is disposed at a position overlapping an outer edge portion 50b of the control board 50 or outside the outer edge portion 50b. In this embodiment, as illustrated in FIG. 3, in the Z-axis direction, an inner end portion of the radiation shielding member 22 overlaps the outer edge portion 50b. Further, as illustrated in FIG. 8, a range R1 in which the heat absorbing member 60 is disposed in the Y-axis direction is included in a range R2 in which the radiation shielding member 22 is disposed in the Y-axis direction. According to this configuration, interference (contact) between the heat absorbing member 60 and the control board 50 can be suitably suppressed. The above effect is more suitably exhibited by disposing the inner end portion of the radiation shielding member 22 outside the outer edge portion 50b. This can facilitate assembly work (for example, work of connecting the end portion 41b of the flexible printed circuit 41 to the connector 51 of the control board 50) and suppress the transfer of heat generated in the IC chip 42 to the control board 50 via the heat absorbing member 60.

Further, as illustrated in FIGS. 2 and 7, the radiation imaging device 1 includes a plurality of flexible printed circuits 41 arranged along the X-axis direction, each having an IC chip 42 mounted thereon. Further, one heat absorbing member 60 extends in the X-axis direction so as to correspond to a plurality of (six in this embodiment) flexible printed circuits 41. That is, in this embodiment, as illustrated in FIG. 7, two (a total of four) heat absorbing members 60 are provided on both side edge portions of the control board 50 in the Y-axis direction. According to this configuration, by providing one heat absorbing member 60 that is long in the X-axis direction for a plurality of (six as an example) IC chips 42 (flexible printed circuits 41) arranged side by side in the X-axis direction, the volume and heat capacity of one heat absorbing member 60 can be more effectively increased. This can more effectively suppress the transfer of heat from the heat absorbing member 60 to the support member 20, that is, the transfer of heat from the heat absorbing member 60 to the sensor panel 30 via the support member 20. Further, as compared with a case where the heat absorbing member 60 is provided individually for each IC chip 42, the number of parts of the heat absorbing member 60 can be reduced, and the work of attaching the heat absorbing member 60 can be facilitated. Further, in a case where the heat absorbing member 60 is fixed to the support member 20 (in this embodiment, the radiation shielding member 22) by adhesion or the like, the fixing stability of the heat absorbing member 60 can also be enhanced.

### [Modifications]

The present disclosure is not limited to the above embodiment. For the material and shape of each component described above, various materials and shapes can be used, not limited to the materials and shapes described above. Further, some of the components included in the radiation imaging device 1 according to the above embodiment may be omitted or changed as appropriate. For example, although some characteristic components included in the radiation imaging device 1 and some effects exhibited by each component have been described in the above embodiment, the radiation imaging device according to the present disclosure does not necessarily need to be configured to exhibit all the effects described in the above embodiment, and may be configured to exhibit only some of the effects described in the above embodiment. In the latter case, the radiation imaging device only needs to have a component essential for exhibiting at least the partial effect, and a component that is not essential for exhibiting the partial effect may be omitted or changed as appropriate. When focusing on one effect, the component essential for exhibiting the one effect should be reasonably grasped based on technical common sense and the description of this specification, with a person skilled in the art as a standard. Hereinafter, some specific modifications of the radiation imaging device of the present disclosure will be exemplified, but it goes without saying that the modifications of the radiation imaging device are not limited to the specific forms exemplified below.

In the above embodiment, the heat absorbing member 60 is provided only for the ROIC (IC chip 42), but it may be provided for an IC chip other than the above (for example, the IC chip 42A constituting the vertical shift register in the above embodiment). Further, for example, the heat absorbing member 60 may be provided only for the IC chip 42A.

In the above embodiment, the detection region R was a region to which an indirect conversion method was applied, in which a radiation image is converted into an optical image by the scintillator 34 and then the optical image is captured by the light receiving unit 32 to obtain an image, but the detection region R may be a region to which a direct conversion method is applied, in which a radiation image is directly captured to obtain an image. For example, on the first surface 31a of the sensor substrate 31, a pixel circuit configured to accumulate and transfer charge may be provided instead of the light receiving unit 32, and a solid material (conversion unit) (for example, CdTe, CdZnTe, GaAs, InP, TlBr, HgI2, PbI2, Si, Ge, and a-Se, etc.) that directly converts radiation into charge may be provided instead of the scintillator 34. As a result, a detection region R to which the direct conversion method is applied is obtained. The detection region R in this case is a region where radiation is incident, and is a region to which a bias voltage is applied (that is, a region to be an object for acquiring an image).

In the above embodiment, the sensor panel 30 in which polycrystalline silicon or amorphous silicon or the like is formed on the sensor substrate 31, which is a glass substrate, has been described, but the sensor panel 30 is not limited to the above configuration, and may have, for example, a configuration in which a light receiving unit is formed on a single crystal silicon substrate. Further, the sensor substrate 31 is not limited to a glass substrate, and may be, for example, a film-like substrate (flexible printed circuit) or the like.

Further, the sensor panel 30 may include a cover member (for example, aluminum or the like) that covers the scintillator 34. Further, although the sensor substrate 31 is adhered to the base plate 21 by a double-sided tape or the like, for example, in a high-temperature and high-humidity environment, if a warping stress of the sensor substrate 31 becomes larger than an adhesive force of the double-sided tape, the sensor panel 30 may float up from the base plate 21 (move toward the top wall 11 side). In order to prevent such floating of the sensor panel 30, a member (for example, urethane sponge or the like) for applying a force to the sensor panel 30 in a direction to suppress the floating when the floating occurs may be disposed between the sensor panel 30 and the top wall 11.

Further, in the above embodiment, the IC chip 42 was provided on a surface on the base plate 21 (top wall 11) side (upper surface in FIG. 3) of the flexible printed circuit 41, but the IC chip 42 may be provided on a surface on a side opposite to the base plate 21 (top wall 11) side (bottom wall 12 side) (lower surface in FIG. 3) of the flexible printed circuit 41. In this case, the capacitor C is also provided on the surface on the side opposite to the base plate 21 (top wall 11) side (bottom wall 12 side) of the flexible printed circuit 41, similarly to the IC chip 42. In the above modification, although the heat absorbing member 60 (heat absorbing portion 61) does not directly contact the IC chip 42, it can absorb the heat generated from the IC chip 42 via a portion of the flexible printed circuit 41 where the IC chip 42 is provided. Therefore, the above modification also exhibits the same effect as the radiation imaging device 1 described above (that is, an effect that heat from the IC chip 42 can be effectively absorbed by the heat absorbing member 60, and heat can be appropriately released from the heat radiating portion 62 into the air). In the above modification, the heat absorbing portion 61 can be defined as "a heat absorbing portion that is disposed in a region overlapping the flexible printed circuit 41 when viewed from the Z-axis direction (first direction) and includes a portion overlapping the mounting surface 421 of the IC chip 42 in the Z-axis direction". Further, the IC chip 42 is disposed on a side opposite to a side where the heat absorbing portion 61 is located with respect to the flexible printed circuit 41. Further, the thickness T1 of the heat absorbing portion 61 in the above modification can be defined in the same manner as in the above embodiment.

### Reference Signs List

- 1: Radiation imaging device
- 20: Support member
- 21: Base plate
- 21a: Support surface
- 21b: Back surface
- 22: Radiation shielding member
- 30: Sensor panel
- 41: Flexible printed circuit
- 41b: End portion (one end)
- 42, 42A: IC chip
- 50: Control board
- 50a: Connection surface
- 50b: Outer edge portion
- 51: Connector (terminal)
- 60: Heat absorbing member
- 61: Heat absorbing portion
- 61a: Contact surface
- 61a1: Inner end portion
- 62: Heat radiating portion
- 421: Mounting surface
- C: Capacitor
- R: Detection region.

## Claims

1. A radiation imaging device comprising:
a sensor panel provided with a detection region for detecting radiation;
a support member having a support surface that supports the sensor panel, and a back surface opposite to the support surface;
a control board disposed at a position facing the back surface of the support member and configured to process a signal read out from the sensor panel;
a flexible printed circuit disposed to connect the sensor panel and the control board through a side of the support member when viewed from a first direction orthogonal to the support surface;
an IC chip mounted on the flexible printed circuit and configured to perform processing for reading out the signal from the sensor panel; and
a heat absorbing member provided between the support member and the IC chip on a side opposite to a side where the sensor panel is located with respect to the support member, and configured to absorb heat from the IC chip,
wherein the heat absorbing member includes:
a heat absorbing portion disposed in a region overlapping the flexible printed circuit when viewed from the first direction, and having a contact surface that contacts a surface of the IC chip opposite to a mounting surface of the IC chip on the flexible printed circuit; and
a heat radiating portion connected to the heat absorbing portion and extending outward beyond an outer edge of the flexible printed circuit in a third direction intersecting a second direction, which is an extending direction of the flexible printed circuit, when viewed from the first direction, and
wherein a thickness of the heat radiating portion in the first direction is greater than a thickness of the heat absorbing portion in the first direction.

2. The radiation imaging device according to claim **1,** wherein the heat absorbing member is not adhered to the flexible printed circuit and the IC chip.

3. The radiation imaging device according to claim 1 or 2, wherein the heat absorbing member includes a plurality of sheet members stacked in the first direction.

4. The radiation imaging device according to any one of claims 1 to 3, wherein the heat absorbing member is disposed outside the detection region when viewed from the first direction.

5. The radiation imaging device according to any one of claims 1 to 4, wherein
a thickness of the heat absorbing member in the first direction is substantially uniform over an entire region of the heat absorbing member in the third direction in a natural state, and
the heat absorbing portion is in a state of being compressed in the first direction by being sandwiched between the flexible printed circuit and the support member.

6. The radiation imaging device according to any one of claims 1 to 5, wherein
one end of the flexible printed circuit is connected to a terminal provided on a connection surface, which is a surface of the control board opposite to a surface facing the back surface,
a capacitor is provided on the flexible printed circuit between the one end and a region where the IC chip is mounted, and
the heat absorbing member is disposed so as not to be in contact with the capacitor.

7. The radiation imaging device according to any one of claims 1 to 6, wherein
one end of the flexible printed circuit is connected to a terminal provided on a connection surface, which is a surface of the control board opposite to a surface facing the back surface,
a capacitor is provided on the flexible printed circuit between the one end and a region where the IC chip is mounted, and
an inner end of the contact surface of the heat absorbing portion closest to the control board is located at a position farther from the back surface than the connection surface of the control board in the first direction, whereby the capacitor is disposed so as not to be in contact with the control board.

8. The radiation imaging device according to any one of claims 1 to 7, wherein
one end of the flexible printed circuit is connected to a terminal provided on a connection surface, which is a surface of the control board opposite to a surface facing the back surface, and
when viewed from the third direction, the contact surface of the heat absorbing portion is inclined so as to become more distant from the back surface along the first direction as it approaches the control board along the second direction.

9. The radiation imaging device according to any one of claims 1 to 8, wherein
the support member includes a base plate having the support surface and the back surface, and a radiation shielding member provided at an edge portion of the base plate when viewed from the first direction, and
the heat absorbing member is provided between the radiation shielding member and the IC chip.

10. The radiation imaging device according to claim 9, wherein a heat capacity of the heat absorbing member is greater than a heat capacity of the radiation shielding member.

11. The radiation imaging device according to claim 9 or 10, wherein
when viewed from the first direction, the radiation shielding member is disposed at a position overlapping an outer edge portion of the control board or outside the outer edge portion, and
a range in which the heat absorbing member is disposed in the second direction is included in a range in which the radiation shielding member is disposed in the second direction.

12. The radiation imaging device according to any one of claims 1 to 11, wherein
a plurality of the flexible printed circuits are arranged along the third direction, each having the IC chip mounted thereon, and
the heat absorbing member extends in the third direction so as to correspond to the plurality of flexible printed circuits.
